# EUROPEAN PATENT APPLICATION

(11) **EP 2 873 973 A1**
(43) Date of publication of application: **20.05.2015**
(21) Application number: 13195397.8
(22) Date of filing: 03.12.2013
(51) Int. Cl.: G01N 33/487

(54) **NFC or RFID based bio sensor measurement device and measuring method using the same**

(30) Priority: 13.11.2013 KR 20130137367
(71) Applicant: Center for Integrated Smart Sensors Foundation, Daejeon (KR)
(72) Inventor: Chong-Min, Kyung, Daejeon (KR); Cho, Hyun Tae, Daejeon (KR); Lee, Seung Ro, Daejeon (KR)
(74) Representative: Gille Hrabal

(57) **Abstract**

Disclosed herein are an NFC or RFID based bio sensor measurement device and a measuring method using the same capable of measuring components by being linked with a wireless communication device such as a smart phone. According to the present invention, the NFC or RFID based bio sensor measurement device includes: a bio sensor unit (100) embedded with a reaction reagent; and an NFC or RFID unit (200) which is electrically connected to the bio sensor unit (100) and wirelessly receives power from the external electronic devices and measures a measurement target value using the bio sensor unit (100) and wirelessly transmits the measured value to the external electronic devices.

## Description

### CROSS REFERENCE TO RELATED APPLICATION

This application claims the benefit of Korean Patent Application No. 10-2013-0137367, filed on Nov. 13, 2013, entitled "NFC OR RFID BASED BIO SENSOR MEASUREMENT DEVICE AND MEASURING METHOD USING THE SAME", which is hereby incorporated by reference in its entirety into this application.

### BACKGROUND OF THE INVENTION

### 1. Technical Field

The present invention relates to a near field communication (NFC) or radio-frequency identification (RFID) based bio sensor measurement device and a measuring method using the same, and more particularly, to an electrochemical sensor measurement device and a measuring method using the same capable of monitoring biological components, such as blood and body fluid of human or animal, by using an NFC or RFID based device, that is, a smart device, such as a smart phone and a smart pad.

### 2. Description of the Related Art

Recently, as modern people are eating western food, people suffering from a so-called adult disease such as diabetes, high cholesterol, and thrombosis are on the rise and young women with iron deficiency anemia have suddenly increased due to excessive dieting A simple method of understanding the relative seriousness of the adult disease is a method of measuring a biological component within a blood. The biological component measurement may figure out the amount of various components included in blood, such as blood sugar, anemia, and blood coagulation, such that an ordinary person may easily determine whether numerical values for specific components are in a normal range or an abnormal range without seeing the doctor.

As one of the simple methods for measuring biological components, there is a method of injecting blood collected at finger tips using a disposable electrochemical bio sensor into a strip and then quantitatively analyzing an output signal using an electrochemical or photometric method, and the method may display the corresponding component amount on a measurement device and therefore may be appropriate for the lay public.

An electrochemical bio sensor according to the related art is disclosed in 'Patent Document 1'.

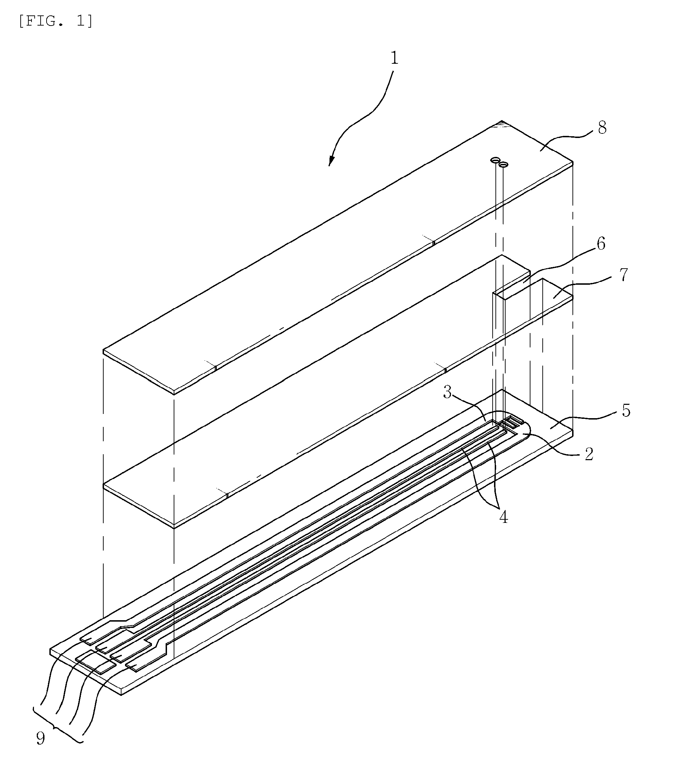

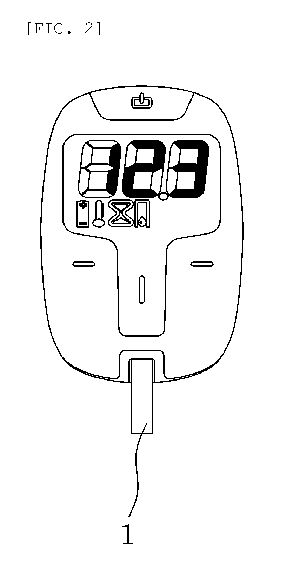

FIG. 1 illustrates the electrochemical bio sensor according to the related art. The electrochemical bio sensor 1 according to the related art is configured to include: a lower plate 5 on which an operation electrode 2, a reference electrode 3, and a specimen recognition electrode 4 are formed; a middle plate 7 provided with a specimen insertion passage 6 and stacked on the lower plate 5; an upper plate 8 stacked on the middle plate 7, in which a connection terminal 9 among the operation electrode 2, the reference electrode 3, and the specimen recognition electrode 4 is inserted into the measurement device to display specific components of blood on the measurement device as illustrated in FIG. 2.

However, the electrochemical bio sensor according to the related art has a problem in that a user separately purchases the measurement device to increase financial burdens and carries the measurement device at the time of movement, such as a business trip and a journey.

### [Related Art Document]

### [Patent Document]

(Patent Document 0001) KR 10-1003077 B1 (December 21, 2010)

### SUMMARY OF THE INVENTION

An object of the present invention is to provide a near field communication (NFC) or radio-frequency identification (RFID) based bio sensor measurement device and a measuring method using the same capable of diagnosing numerical values of biological components using a smart device embedded with the NFC or RFID function without a separate measurement device.

According to an exemplary embodiment of the present invention, there is provided an NFC or RFID based bio sensor measurement device, including: a bio sensor unit embedded with a reaction reagent; and an NFC or RFID unit which is electrically connected to the bio sensor unit, wirelessly receives power from an external electronic device, measures a measurement target value using the bio sensor unit, and wirelessly transmits the measured value to the external electronic device.

According to an exemplary embodiment of the present invention, there is provided a measuring method using an NFC or RFID based bio sensor measurement device, including: approaching an external electronic device to the NFC or RFID based bio sensor measurement device as described above; starting, by the external electronic device, measurement to carry out a start command; supplying power to the NFC or RFID based bio sensor measurement device and transmitting a command thereto, by the external electronic device; generating, by the NFC or RFID based bio sensor measurement device, power by receiving the command; recognizing a command and applying a voltage or a current to a sensor to, by the NFC or RFID based bio sensor device; converting, by the NFC or RFID based bio sensor device, an analog signal into a digital signal; storing the converted signal in a memory or transmitting the converted signal to the external electronic device, by the NFC or RFID based bio sensor measurement device; receiving the converted signal and displaying the received signal using embedded applications, by the external electronic device; and turning off an NFC function of the external electronic device.

### BRIEF DESCRIPTION OF THE DRAWINGS

FIG. 1 is an exploded perspective view of an electrochemical bio sensor according to the related art.
FIG. 2 is a measurement device which is coupled with the electrochemical bio sensor according to the related art.
FIG. 3 is a concept diagram of NFC or RFID based bio sensing according to an exemplary embodiment of the present invention.
FIG. 4 is a perspective view of an NFC or RFID based bio sensor measurement device according to an exemplary embodiment of the present invention.
FIG. 5 is an internal configuration diagram of an NFC or RFID based bio sensor measurement device according to an exemplary embodiment of the present invention.
FIG. 6 is a configuration diagram of an RF interface according to an exemplary embodiment of the present invention.
FIG. 7 is a diagram illustrating the NFC or RFID based bio sensor measurement device according to the exemplary embodiment of the present invention in which a measurement processor is included.
FIGS. 8 and 9 are diagrams illustrating an NFC or RFID based bio sensor measurement device according to another exemplary embodiment of the present invention.
FIG. 10 is a use state diagram of a reader or a smart device.
FIG. 11 is a flow chart of a measuring method using the NFC or RFID based bio sensor measurement device according to an exemplary embodiment of the present invention.

### DESCRIPTION OF THE EXEMPLARY EMBODIMENTS

Hereinafter, an NFC or RFID based bio sensor measurement device and a measuring method using the same according to an exemplary embodiment of the present invention will be described in more detail with reference to the accompanying drawings.

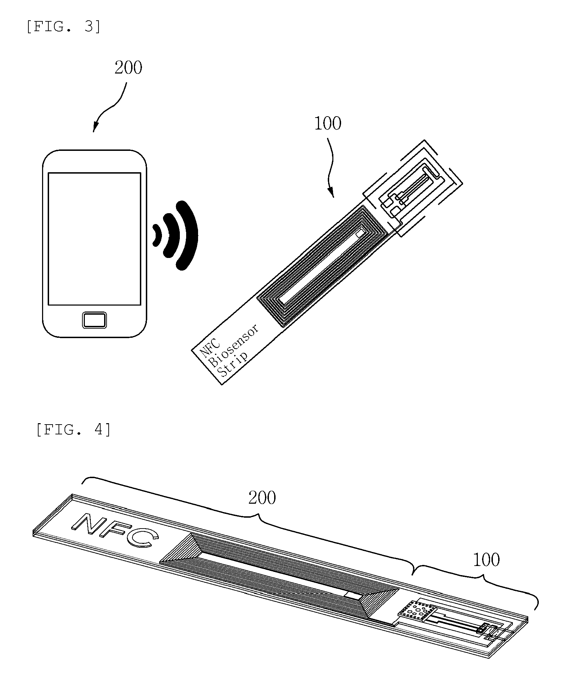

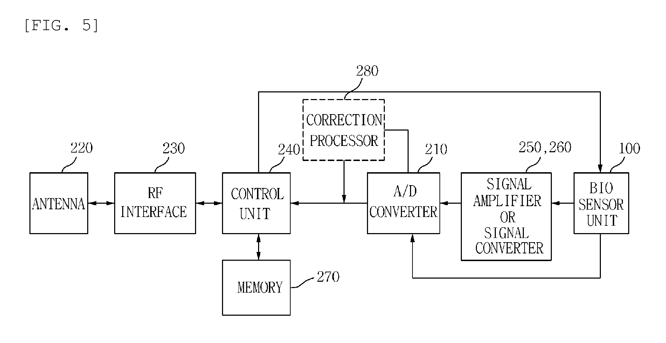

FIG. 3 is a concept diagram of NFC or RFID based bio sensing according to an exemplary embodiment of the present invention, FIG. 4 is a perspective view of an NFC or RFID based bio sensor measurement device according to an exemplary embodiment of the present invention, and FIG. 5 is an internal configuration diagram of an NFC or RFID based bio sensor measurement device according to an exemplary embodiment of the present invention.

A basic technical idea of the present invention is to display a measurement value sensed by an NFC or RFID based bio sensor measurement device 100 on a personal smart device 200 which is spread to most people in advance without purchasing a separate measurement device, as illustrated in FIG. 3. The NFC or RFID based bio sensor measurement device according to the exemplary embodiment of the present invention may wirelessly receive power from external electronic devices by the NFC or RFID scheme, etc., and may transmit and receive data and commands, and therefore needs not include a separate battery.

The NFC or RFID based bio sensor measurement device 100 according to the exemplary embodiment of the present invention is configured to include: a bio sensor unit 100 embedded with a reaction reagent which has physical properties changed by reacting with a material such as blood; and an NFC or RFID unit 200 which is electrically connected to the bio sensor unit 100 and wirelessly receives power from the external electronic devices and measures a measurement target value using the bio sensor unit 100 and wirelessly transmits the measured value to the external electronic devices. An example of the external electronic device may include a smart device or a reader. Herein, the smart device which is widely spread and conveniently carried and may install applications suitable for the NFC or RFID based bio sensor measurement device is preferable.

The bio sensor unit 100 is a component which measures a change in electrochemical characteristics of a specimen by reaction of the specimen, and is fixed with the reaction reagent, such as an enzyme and a specific polymer material, which selectively reacts with a biological specimen to be able to measure a physical signal or a chemical signal.

The bio sensor unit 100 is embedded with a reaction reagent which may be appropriate for reacting with each marker compound for measurement of blood sugar, measurement of anemia, measurement of blood coagulation time, and the like.

Typically, the bio sensor is configured to include a pair of measurement electrodes or additionally include a specimen recognition electrode (when one of the measurement electrodes is used as a common electrode, one specimen recognition electrode is used and otherwise a pair of specimen recognition electrodes is used), such that the number of electrode terminals of the bio sensor unit 100 is formed in two to four.

The NFC or RFID unit 200 is a component which is electrically connected to the bio sensor unit 100 to measure the measurement target value, process data, transmits and receives data to and from the external electronic devices, and receives power from the external electronic devices and includes an A/D converter 210 which converts an analog signal received from the bio sensor unit 100 into a digital signal, an antenna 220 which transmits and receives data and power to and from the external electronic devices, an RF interface 230 which senses or modulates and demodulates data or control signals received from the external electronic devices through the antenna 220, and a control unit 240 which processes commands (commands using the NFC) received from the external electronic devices.

Further, the NFC or RFID unit 200 may be configured to further include: a signal amplifier 250 amplifying a magnitude in signal received from the bio sensor unit 100; a signal converter 260 converting the signal received from the bio sensor unit 100 into a signal appropriately processed by the control unit 240; an electrically programmable and erasable memory 270; a correction processor 280 for correcting a signal; a measurement processor 290 for measuring the measurement target value based on the signal received from the bio sensor unit 100.

The A/D converter 210 is a component converting the analog signal into the digital signal and is connected to the bio sensor unit 100 to convert the analog signal received from the bio sensor unit 100 into the digital signal and transmit the converted signal to the control unit 240.

The antenna 220 is a component which may transmit and receive data and power to and from the external electronic devices such as a smart device and is formed to have a predetermined pattern as illustrated in FIG. 4 as an example. The detailed specifications (line width, pattern, and the like) of the antenna 220 vary depending on a frequency, a distance of arrival, energy transmission per time, and the like in the NFC or RFID, which are general design matters to those skilled in the art and therefore may be appropriately designed to meet the matters.

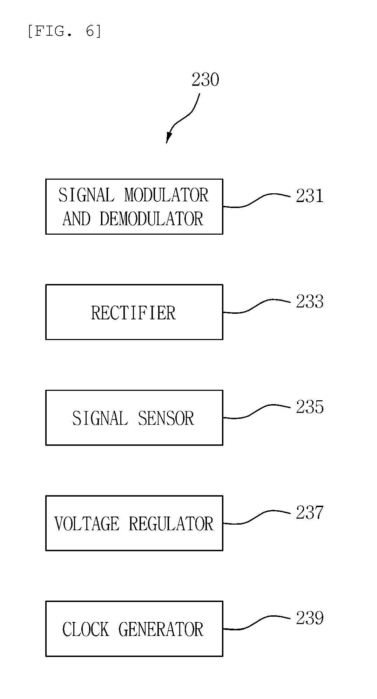

FIG. 6 is a configuration diagram of an RF interface according to an exemplary embodiment of the present invention.

The RF interface 230 is a component which senses and modulates and demodulates the signal received through the antenna 220 and is connected to the antenna 220 and configured to include a signal modulator and demodulator 231 and a rectifier 233.

The signal modulator and demodulator 231 is a component which modulates a signal including the measured data into a waveform suitable for transmission so as to transmit the measured data to the external electronic devices or demodulates the signals received from the external electronic devices into a waveform appropriately processed by the control unit 240 and transmits the signal modulated into several bands depending on the current NFC or RFID standard to the antenna 220.

The rectifier 233 is a component which receives power from the external electronic devices to generate power required to drive the signal modulator/demodulator 231, the control unit 240, and the like.

A signal sensor 235 is a component which senses the signals received from the external electronic devices through the antenna 220 and transmits the sensed signals to the signal modulator and demodulator 231 or the control unit 240. Due to the signal modulator and demodulator 231 and the signal sensor 235, the NFC or RFID between the NFC or RFID based bio sensor measurement device according to the exemplary embodiment of the present invention and the external electronic devices may be made.

Further, the RF interface 230 is configured to further include a voltage regulator 237 which regulates power generated from the rectifier 233 to a required voltage and a clock generator 239 which generates a clock to rectify the signal or generate the clock and supply the signal or clock to the control unit 240. In most devices, a power generation unit is configured to include the rectifier 233 and the voltage regulator 237.

Since the currently standardized NFC has a frequency band of 13.56 MHz, and a maximum operation distance of about 20 cm or so, generally, about 10 cm, the external electronic devices need to approach the NFC or RFID based bio sensor measurement device according to the exemplary embodiment of the present invention (in the case of the RFID, a frequency band or an operation distance may vary). When the external electronic devices approach the NFC or RFID sensor measurement device, an electromagnetic induction phenomenon occurs from the external electronic devices to the antenna 220. In this case, the rectifier 233 appropriately converts power transferred by the electromagnetic induction by the voltage regulator and then supplies the converted power to devices in the sensor measurement device.

The control unit 240 is a component which recognizes and processes the commands transmitted from the external electronic devices and is connected to the RF interface 230 to recognize the commands transmitted from the external electronic device through the antenna 220 and applies a current or a voltage to the bio sensor unit 100. The NFC or RFID based bio sensor measurement device according to the exemplary embodiment of the present invention is a passive type and therefore uses the power generated from the rectifier 233.

The signal amplifier 250 is a component which amplifies a magnitude of a signal and when a signal transmitted from the bio sensor unit 100 is weak, amplifies the signal to easily process the signal. The signal amplifier may be omitted when the performance of the control unit 240 is good or the magnitude of the signal transmitted from the bio sensor unit 100 is sufficiently large.

The signal converter 260 is a component which converts the signal transmitted from the bio sensor unit 100 into a signal appropriately processed by the control unit 240, for example, converts a current into a voltage. Generally, since electronic devices often use a voltage value as an input, the signal converter 260 which converts a current value into a voltage value is generally used, in which an example of the signal converter 260 may include ICs such as an OP AMP. The signal converter 260 may be omitted when the devices in the NFC or RFID based bio sensor measurement device is designed to process the current value. That is, when the current value is used as the input of the sensor, the voltage value may be obtained as a result and therefore the signal converter 260 may be omitted.

The memory 270 is a component which stores data processed by the control unit 240 and may be preferably an electrically erasable and programmable read only memory (EEPROM) which is connected to the control unit 240 to carry out an electrically programmable and erasable function. The EEPROM, which is a nonvolatile memory which stably stores data for a long period of time without a power supply, is a modification of an erasable and programmable read only memory (EPROM) and may electrically erase and rewrite recorded data. The data processed by the control unit 240 are stored in the EEPROM or may be transmitted to the external electronic devices without the above storage process. When there is no need to store the data processed by the control unit 240, the memory 270 may be omitted.

The NFC or RFID based bio sensor measurement device may further include the correction processor 280. The correction processor 280 is a component which corrects the data measured by the bio sensor unit 100 among the digital signal transmitted from the A/D converter 210 and is connected to the A/D converter 210 to correct the data measured by the bio sensor unit 100 and transmit the corrected data to the control unit 240. When the correction processor 280 is used, power consumption is increased but the data measured by the bio sensor unit 100 may be corrected by more precise and accurate calculation. The data correction function may be designed to be processed by the control unit 240.

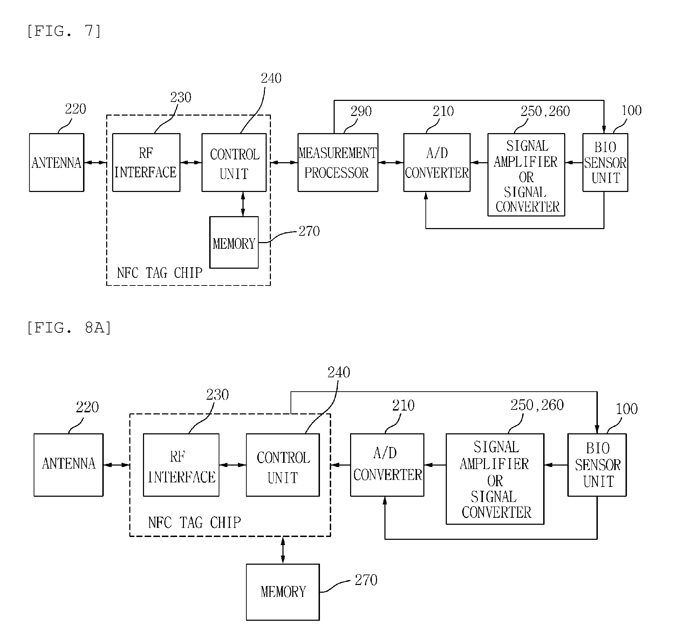

If necessary, the NFC or RFID based bio sensor measurement device may be configured to further include the measurement processor. FIG. 7 is a diagram illustrating the NFC or RFID based bio sensor measurement device according to the exemplary embodiment of the present invention in which the measurement processor is included. The measurement processor 290 is a component which is required to implement the NFC or RFID based bio sensor measurement device according to the exemplary embodiment of the present invention using the generally commercialized NFC tag chip. In this case, since the general NFC tag chip is involved in only the NFC communication and may not extract the measurement target value such as blood sugar level from the signal measured by the bio sensor unit 100, the measurement processor 290 is required to understand the measuring target value. The measurement processor 290 receives the signal from the A/D converter 210 (when there is the correction processor, receives the corrected signal from the correction processor) to measure the measurement target value and then transmit the measured value to the control unit 240.

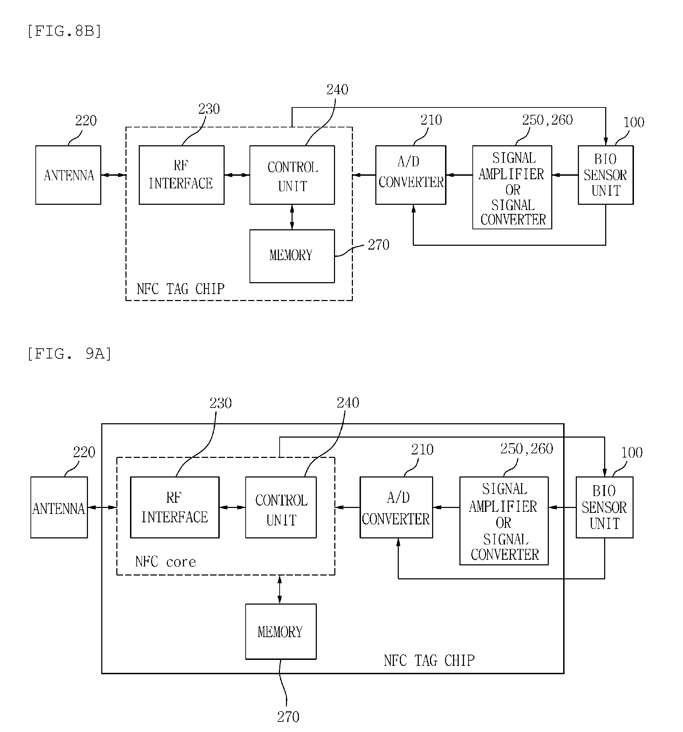

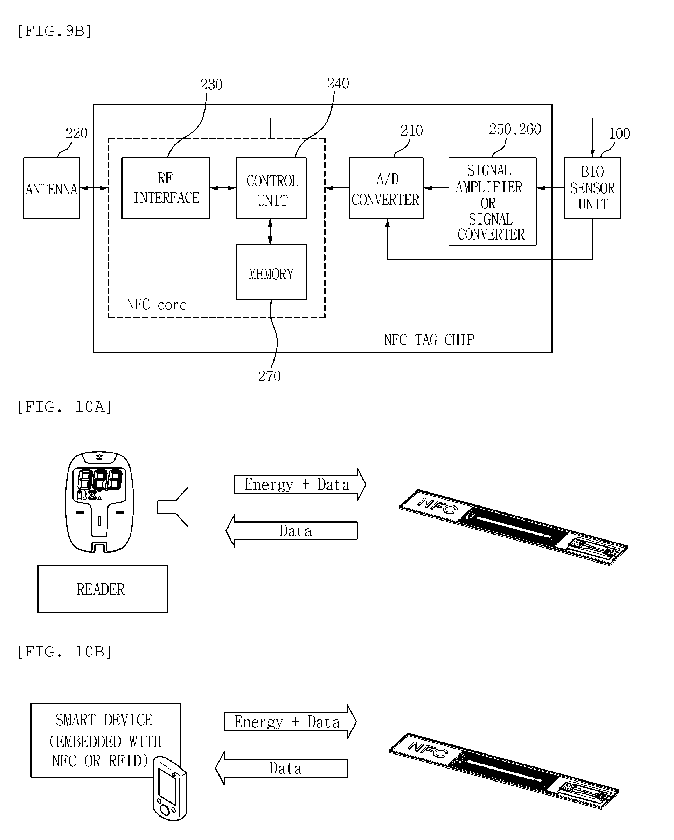

FIGS. 8 and 9 are diagrams illustrating an NFC or RFID based bio sensor measurement device according to another exemplary embodiment of the present invention. As illustrated in FIG. 8A, the RF interface 230 and the control unit 240 are formed as the NFC tag chip, or as illustrated in FIG. 8B, the RF interface 230, the control unit 240, and the memory 270 may be formed as the NFC tag chip. Further, as illustrated in FIG. 9, the NFC core including the RF interface 230 and the control unit 240 is formed and the NFC core may also be formed with the NFC tag chip, which is coupled with the A/D converter 210, the signal amplifier 250, and the signal converter 260, in a system on chip (SoC) type.

FIG. 10 is a use state diagram of a reader or a smart device. As illustrated in FIG. 10A, the measured value may be displayed by the reader or as illustrated in FIG. 10B, the measured value may also be displayed by the smart device.

An example of the smart device may preferably include a smart phone, a PDA, a tablet PC, a notebook, and the like in which the NFC/RFID function is embedded.

The reader means a device which is embedded with the NFC/RFID function to communicate with the NFC or RFID based bio sensor measurement device according to the exemplary embodiment of the present invention so as to read the measured data. Therefore, a user which does not use the smart device requires the reader.

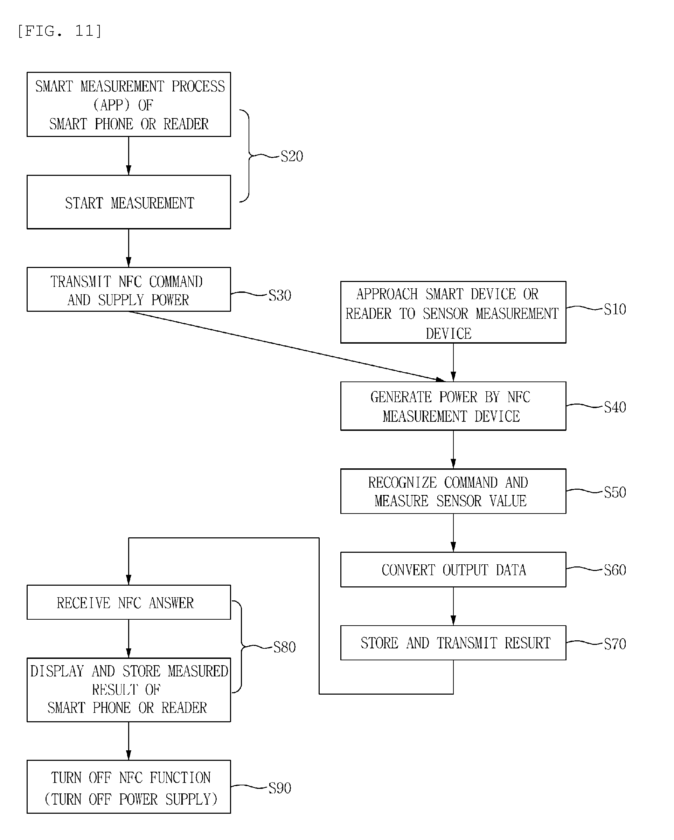

Next, a measuring method using the NFC or RFID based bio sensor measurement device according to an exemplary embodiment of the present invention will be described. FIG. 11 is a flow chart of a measuring method using the NFC or RFID based bio sensor measurement device according to an exemplary embodiment of the present invention.
① Approaching Step (S10): The external electronic devices approach the NFC or RFID based sensor measurement device according to the exemplary embodiment of the present invention.
② Measurement Starting Step (S20): The step is a step of carrying out a start command from the external electronic devices. For example, the start command may be carried out using applications which are installed in the smart device or the start command may be carried out by pressing a button of the reader.
③ Command Transmitting Step (S30): The external electronic devices apply the power to the NFC or RFID based bio sensor measurement device according to the exemplary embodiment of the present invention and transmit the commands.
④ Power Generating Step (S40): The NFC or RFID based bio sensor measurement device according to the exemplary embodiment of the present invention receives the commands through the antenna 220 and generates the power from the rectifier 233.
⑤ Command Recognition Step (S50): The control unit 240 recognizes the commands and acquires the signals by applying a voltage or a current to the bio sensor unit 100.
⑥ Signal Converting Step (S60): The A/D converter 210 converts the analog signal into the digital signal.
⑦ Storing and Transmitting Step (S70): The converted signal is stored in the memory 270 or is transmitted to the external electronic devices through the antenna 220.
⑧ Receiving and Displaying Step (S80): The external electronic devices receive the converted signals and display the received signals using the embedded applications.
⑨ Ending Step (S90): The NFC function of the external electronic devices is turned off to stop the power supply and ends the measurement of the bio sensor unit 100.

The measurement starting step (S20) and the command transmitting step (S30) may be carried out prior to the sensor connecting step (S10). That is, the start command may be carried out by the external electronic devices before the external electronic devices approach the NFC or RFID based bio sensor measurement device according to the exemplary embodiment of the present invention.

According to the exemplary embodiments of the present invention, the NFC or RFID based bio sensor measurement device may have the excellent economical efficiency since the user needs not purchase the separate measurement device and individually purchases the strip one by one. Further, since most of modern people carry a mobile smart device which is an individual necessity, even when the user does not carry the measurement device by mistake when traveling, blood sugar, anemia, blood coagulation time, and the like may be diagnosed on the spot.

## Claims

1. An NFC or RFID based bio sensor measurement device, comprising:
a bio sensor unit embedded with a reaction reagent; and
an NFC or RFID unit which is electrically connected to the bio sensor unit, wirelessly receives power from an external electronic device, measures a measurement target value using the bio sensor unit, and wirelessly transmits the measured value to the external electronic device.

2. The NFC or RFID based bio sensor measurement device of claim 1, wherein the bio sensor unit measures a physical signal or a chemical signal.

3. The NFC or RFID based bio sensor measurement device of claim 1, wherein the NFC or RFID unit carries out NFC or RFID with the external electronic device.

4. The NFC or RFID based bio sensor measurement device of claim 1, wherein the external electronic device is at least any one of a reader, a smart phone, a PDA, a tablet PC, and a notebook.

5. The NFC or RFID based bio sensor measurement device of claim 1, wherein the NFC or RFID unit includes:
an A/D converter which is connected to the bio sensor unit to convert an analog signal received from the bio sensor unit into a digital signal;
an antenna which transmits and receives data and power to and from the external electronic device;
an RF interface which senses or modulates and demodulates data or control signals received from the external electronic device through the antenna; and
a control unit which processes a command received from the external electronic device.

6. The NFC or RFID based bio sensor measurement device of claim 5, further comprising:
a measurement processor which receives the signal from the A/D converter to measure the measurement target value and transmit the measured value to the control unit.

7. The NFC or RFID based bio sensor measurement device of claim 5, wherein the RF interface and the control unit are formed as an NFC tag chip.

8. The NFC or RFID based bio sensor measurement device of claim 5, wherein an NFC core including the RF interface and the control unit is formed, and the NFC core is formed with the NFC tag chip, which is coupled with the A/D converter, a signal amplifier, and a signal converter, in a system on chip (SoC) type.

9. A measuring method using an NFC or RFID based bio sensor measurement device, comprising:
approaching an external electronic device to the NFC or RFID based bio sensor measurement device of claim 1;
starting, by the external electronic device, measurement to carry out a start command;
supplying power to the NFC or RFID based bio sensor measurement device and transmitting a command thereto, by the external electronic device;
generating, by the NFC or RFID based bio sensor measurement device, power by receiving the command;
recognizing a command and applying a voltage or a current to a sensor to acquire a signal, by the NFC or RFID based bio sensor device;
converting, by the NFC or RFID based bio sensor device, an analog signal into a digital signal;
storing the converted signal in a memory or transmitting the converted signal to the external electronic device, by the NFC or RFID based bio sensor measurement device; and
receiving the converted signal and displaying the received signal using embedded applications, by the external electronic device.

10. The measuring method of claim 9, wherein the acquired signal is a physical signal or a chemical signal.

11. The measuring method of claim 9, wherein the command is transmitted and received by NFC or RFID.

12. The measuring method of claim 9, wherein a measurement target value is measured based on the digital signal.
